# EUROPÄISCHE PATENTSCHRIFT

(11) **EP 0 678 518 B1**
(45) Veröffentlichungstag und Bekanntmachung des Hinweises auf die Patenterteilung: **11.03.1998**
(21) Anmeldenummer: 95105544.1
(22) Anmeldetag: 12.04.1995
(51) Int. Cl.: C07D 317/72

(54) **Verfahren zur Herstellung von Zwischenprodukten für die Synthese von Glucose-6-Phosphatase-Inhibitoren sowie neue Zwischenprodukte**
Process for preparation of intermediates for the synthesis of glucose-6-phosphatase inhibitors as well as intermediates
Procédé pour la préparation de produits intermédiaires pour la synthèse des inhibiteurs de glucose-6-phosphatase et produits intermédiaires

(30) Priorität: 18.04.1994 DE 4413402
(43) Veröffentlichungstag der Anmeldung: 25.10.1995
(73) Patentinhaber: HOECHST AKTIENGESELLSCHAFT, 65929 Frankfurt am Main (DE)
(72) Erfinder: Hemmerle, Horst, Dr., D-64653 Lorsch (DE)

(56) Entgegenhaltungen:
- EP-A- 0 587 088

## Beschreibung

EP-A-0 587 088 betrifft substituierte Cyclohexan-Derivate, die das Glucose-6-Phosphatase-System der Leber hemmen. Die Verbindungen eignen sich daher zur Behandlung von Krankheiten, die mit einer erhöhten Aktivität des Glucose-6-Phosphatase-Systems verbunden sind. Die Cyclohexan-Derivate gemäß EP-A-0 587 088 weisen eine Reihe von Stereozentren auf.

Es wurde nun ein Verfahren gefunden, das es ermöglicht, bestimmte Cyclohexan-Derivate gemäß EP-A-0 587 088 in enantiomerenreiner Form herzustellen.

Die Erfindung betrifft daher ein Verfahren zur Herstellung einer Verbindung der Formel I 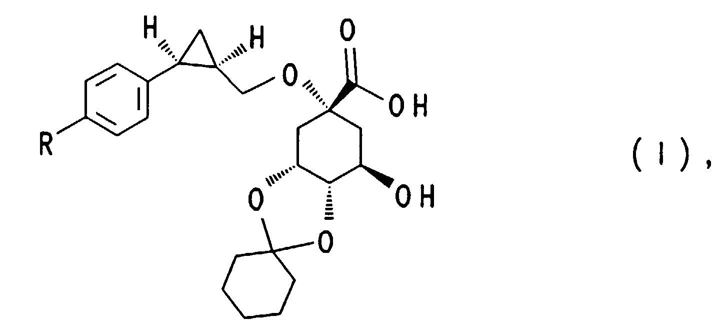 worin R Fluor, Chlor oder Methyl bedeutet, dadurch gekennzeichnet, daß man den Lactonring von 1,2-O-Cyclohexyliden-3,5-lactonyl-cyclohexan-1,2-diol der Formel 1 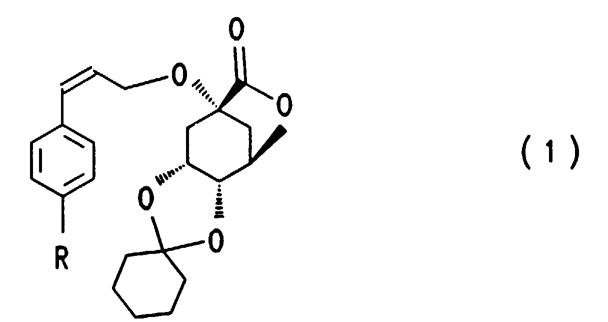 durch Basenkatalyse unter Bildung eines Alkohols der Formel 2 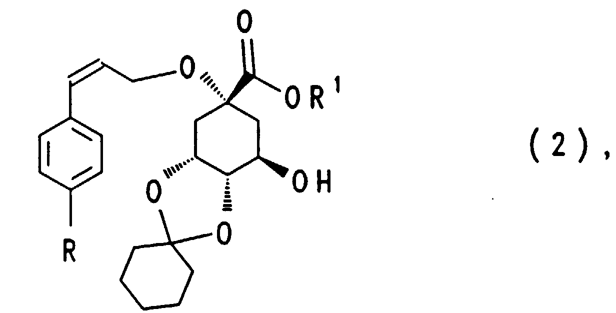 worin R die zu Formel I angegebene Bedeutung hat und R¹ C₁-C₁₀-Alkyl (geradkettig oder verzweigt) oder Benzyl bedeutet, öffnet,
einen erhaltenen Alkohol der Formel 2 mit einem Säurechlorid in Gegenwart einer Base umsetzt zu einem Ester der Formel 3 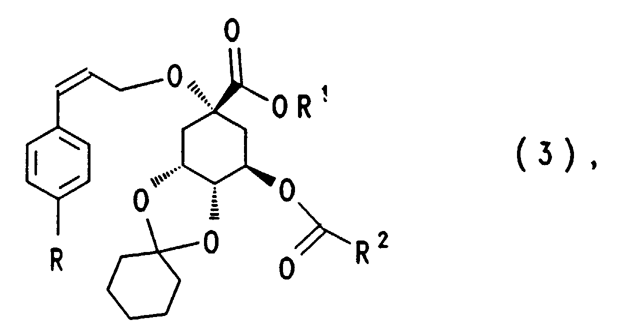 worin R die zu Formel I und R¹ die zu Formel 2 angegebenen Bedeutungen haben und
R² C₁-C₁₀-Alkyl (geradkettig oder verzweigt), Phenyl oder Naphthyl ist, und eine erhaltene Verbindung der Formel 3 mit einem Carben umsetzt zu einem Diastereomerengemisch der Cyclopropylderivate der Formeln 4A und 4B 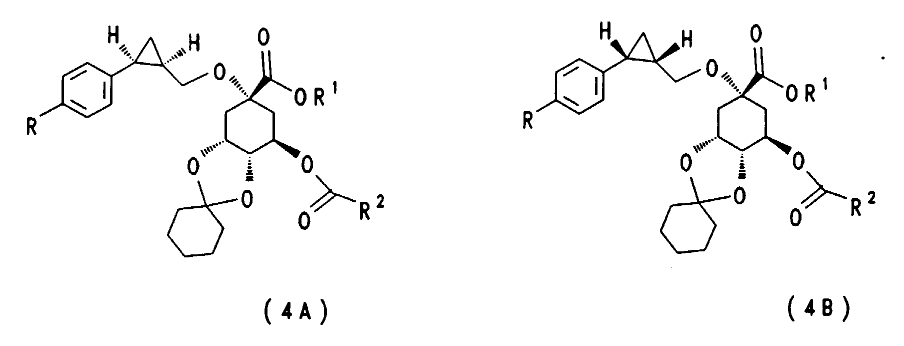 worin R, R¹ und R² die zu den Formeln I, 2 und 3 angegebenen Bedeutungen haben und wobei 4A in deutlichem Überschuß entsteht,
durch Kristallisation eine Verbindung der Formel 4A aus dem Gemisch isoliert und durch alkalische Hydrolyse und anschließendes Ansäuern in eine die Verbindung der Formel I überführt.

Das Verfahren wird vorzugsweise wie folgt durchgeführt:

In der ersten Stufe wird der Lactonring des Lactons 1 (vgl. EP-A- 0 587 088, Beispiel 68A) durch Basenkatalyse in einem Alkohol (C₁-C₁₀-Alkanol, Benzylalkolhol) bei 0°C bis zum Siedepunkt des Lösungsmittels zu 2 geöffnet (bevorzugt ist der Temperaturbereich 0 - 25°C). Um die Löslichkeit von 1 zu erhöhen, können inerte Lösungsmittel wie Tetrahydrofuran oder Dioxan zugesetzt werden. Als Base dient das Alkali-Alkoholat des Alkohols, mit dem die Öffnung des Lacton 1 zu 2 erfolgen soll, bevorzugt sind die Natrium- bzw. die Kaliumalkoholate.

In der 2. Stufe wird der Alkohol 2 mit einem Säurechlorid (R² bevorzugt = C₁-C₁₀-Alkyl, Phenyl) in einem inerten, aprotischen Lösungsmittel, bevorzugt ist Dichlormethan bei -20°C bis zum Siedepunkt des Lösungsmittels in Gegenwart einer Base wie Triethylamin, Ethyldiisopropylamin oder Pyridin und zusätzlich Dimethylaminopyridin zu 3 umgesetzt, bevorzugt ist Triethylamin.

In der 3. Stufe wird das Alken (Ester) 3 in einem inerten Lösungsmittel wie Dichlorethan, Dichlormethan oder Toluol gelöst und bei -20 bis -40°C zu einer Reagenzlösung von Diiodmethan / Diethylzink oder Chloriodmethan / Diethylzink in einem inerten Lösungsmittel, das bereits oben genannt wurde, gegeben. Man erhält eine Mischung von 4A und 4B, wobei 4A in deutlichem Überschuß (4A:4B ca. 5:1) entsteht: Durch übliche Kristallisation läßt sich 4A mit einem Diastereomerenanteil > 98% erhalten.

Durch alkalische Hydrolyse und anschließendes Ansäuern mit Säuren, wie Salzsäure oder Essigsäure, erhält man in der 4. Stufe aus 4A die Verbindung der Formel I.

Der Vorteil gegegenüber dem in EP-A-0 587 088 beschriebenen Verfahren besteht in der Umkehr der Diastereomerenverhältnisse der Cyclopropylseitenkette.
Während bei dem Verfahren gemäß EP-A-0 587 088 das 1S,2R-Cyclopropan 5A (R = Chlor) mit einer Selektivität von etwa 4:1 entsteht, 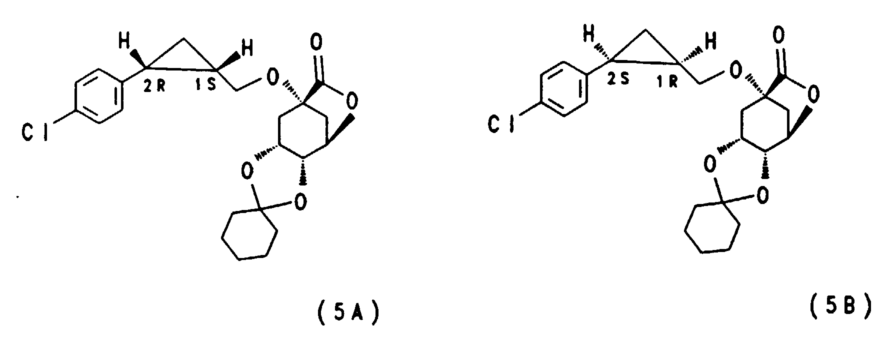 erhält man beim erfindungsgemäßen Verfahren 4A und 4B mit einer Selektivität von 5:1.

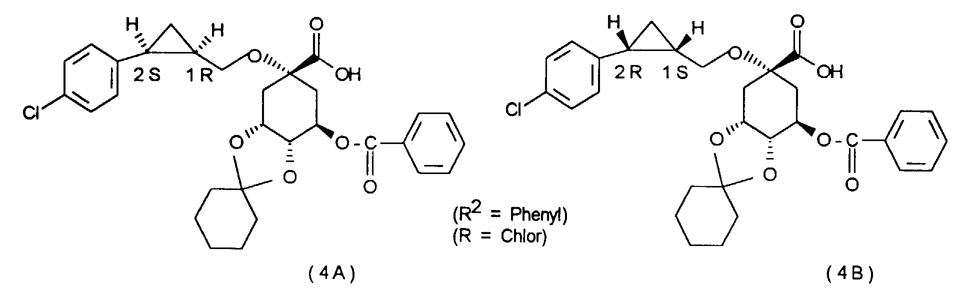

Die Verbindung der Formel I ist ein wertvolles Zwischenprodukt zur Herstellung von Glucose-6-Phosphatase-lnhibitoren gemäß EP-A-0 587 088. Die weitere Umsetzung von Verbindung I zu den Cyclohexanderivaten gemäß EP-A-0 587 088 erfolgt z.B. analog der in dieser Schrift beschriebenen Methode A.

Die aus Verbindung I zugänglichen Glucose-6-Phosphatase-Inhibitoren sind etwa 10fach wirksamer als die aus Verbindung 5A hergestellten Inhibitoren. Mit Hilfe der Verbindung I ist es erstmals gelungen, das wirksamere Diastereomer einer Verbindung gemäß EP-A-0 587 088 diastereoselektiv herzustellen.

Die Verbindungen I sowie die Verbindungen 2, 3 und 4A sind in EP-A-0 587 088 nicht vorbeschrieben. Die Erfindung betrifft daher auch diese Verbindungen. Sie stellen alle wertvolle Zwischenprodukte für die Synthese von Cyclohexan-Derivaten z.B. gemäß EP-A-0 587 088 dar.

### Beispiel 1

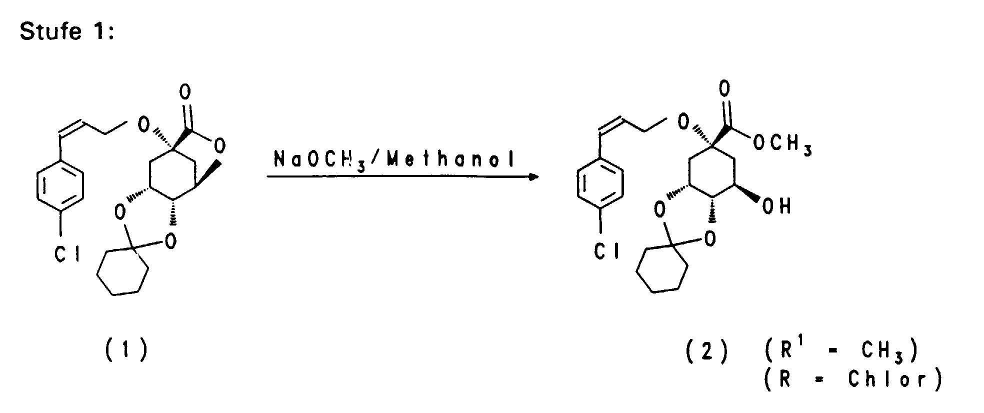

Zu einer Lösung von 5 l wasserfreiem Methanol und 1.5 l wasserfreiem Tetrahydrofuran wurde unter Argon-Atmosphäre bei Raumtemperatur 2.95 g (0.0985 Mol) NaH, 80%ig, portionsweise zugegeben. Anschließend gab man ebenfalls bei Raumtemperatur Verbindung 1 (vergl. EP-A-0 587 088) als Feststoff zu. Nach 3-4 Stunden erhielt man eine klare Lösung. Zur Aufarbeitung gab man 6.0 g Eisessig (pH∼5) und danach portionsweise 2 l Wasser zu. Es bildete sich ein flockiger Niederschlag von nicht umgesetztem Lacton, der sich problemlos abfiltrieren ließ. (Rückgewinnung von Edukt!)
Anschließend engte man das Filtrat solange ein, bis sich ein dicker weißer Niederschlag bildete. Man kühlte mit Eis ab, saugte den Niederschlag ab und wusch mit eiskaltem Methanol/Wasser 1:1 nach.
Nach Trocknen des Niederschlags bei 1 mbar und 40°C erhielt man 370 g (86%) Verbindung 2 als farblosen Feststoff.
Fp.: 102-104°C

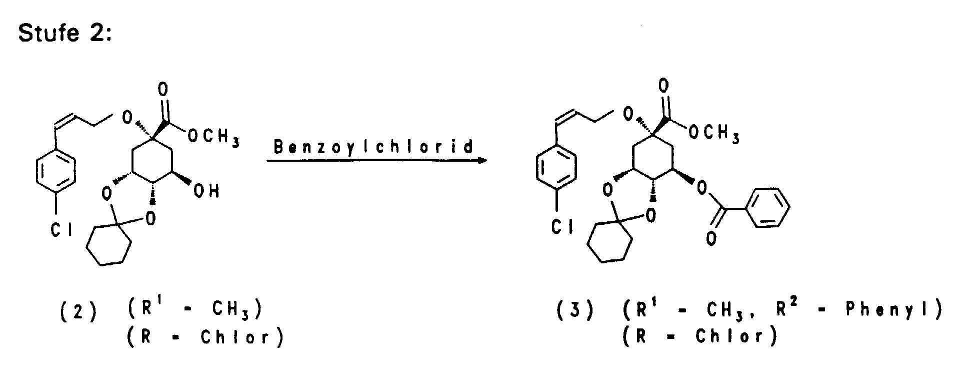

384.0 g (0.879 Mol) Alkohol 2 aus Stufe 1 wurden in 1.3 l wasserfreiem Dichlormethan gelöst. Anschließend wurden 10.74 g (0.0879 Mol) 4-Dimethylaminopyridin sowie 364.8 ml (2.637 Mol) wasserfreies Triethylamin zugegeben. Man kühlte die Lösung auf 0-10°C ab und tropfte 164.7 ml (1.418 Mol) Benzoylchlorid gelöst in 350 ml wasserfreiem Dichlormethan zu. Nach 4 Stunden bei Raumtemperatur waren nur noch Spuren Edukt vorhanden. DC: Essigester/Cyclohexan 1:2
Man gab das Reaktionsprodukt auf 1.5 l Wasser / 400 g NH4Cl / 1 l Eis.
Anschließend extrahierte man zweimal mit Dichlormethan, wusch einmal mit gesättigter Bicarbonat-Lösung und trocknete die vereinigten organischen Phasen mit Na₂SO₄. Nach dem Einengen wurde der Rückstand aus Isopropanol kristallisiert.
Man erhielt 437.0 g (91.9 %) Produkt 3.
Fp.: 104-107°C

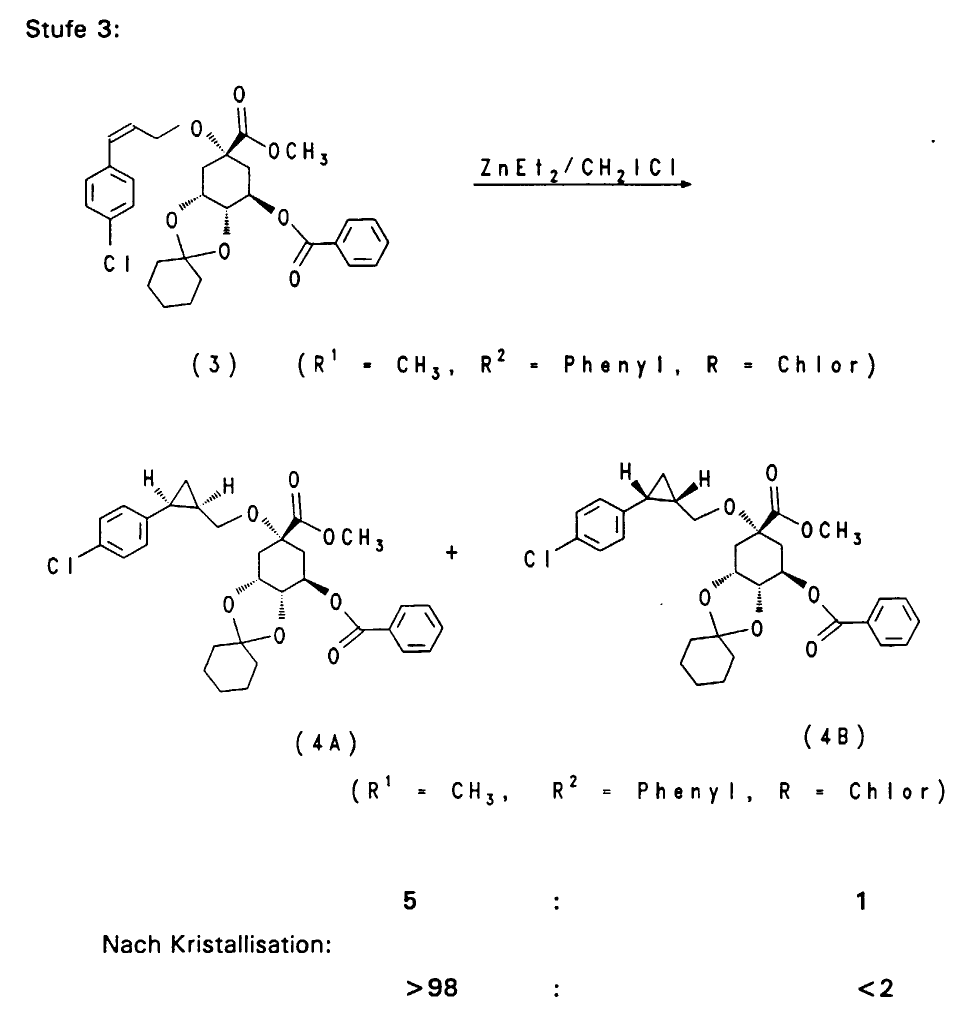

Zu 2 l wasserfreiem Dichlorethan ** wurden bei 0°C unter Argon-Atmosphäre 80.5 ml (0.785 Mol) reines Diethylzink aus Stahlbombe mit Hilfe einer Stahlkanüle übergedrückt.
Anschließend tropfte man bei 0-10°C 114.4 ml (1.57 Mol) Chloriodmethan zu, rührte die entstandene Suspension 30 Minuten nach und tropfte danach 169.89 g (0.314 Mol) des Olefins 3 aus Stufe 2, gelöst in 500 ml wasserfreiem Dichlorethan, zu. Nach 1 Stunde bei 0-10°C ließ man das Reaktionsgemisch auf Raumtemperatur aufwärmen und rührte weitere 3 Stunden bei Raumtemperatur.

Das Reaktionsgemisch wurde unter Stickstoff-Atmosphäre langsam in eine Lösung von 300 g NH4Cl / 1.5 l Eiswasser gegossen und mit Dichlormethan extrahiert. Die vereinigten organischen Phasen wurden mit gesättigter NaCl-Lösung ausgeschüttelt und mit Na₂SO₄ getrocknet.
Anschließend engte man im Vakuum den größten Teil des Lösungsmittels ab und verdünnte mit Isopropanol. Danach engte man weiter ein, bis ein dicker Niederschlag ausfiel. Man saugte diesen Niederschlag ab und kristallisierte zweimal aus Isopropanol um und erhielt 109.8 g (63%) Verbindung 4A mit einem DE > 98 %.
Fp.: 143-144°C
* statt reinem Diethylzink kann auch 1 molare Lösung in Toluol (Aldrich) verwendet werden.
** statt Dichlorethan wurden auch andere inerte Lösungsmittel verwendet (Dichlormethan, Toluol, THF). 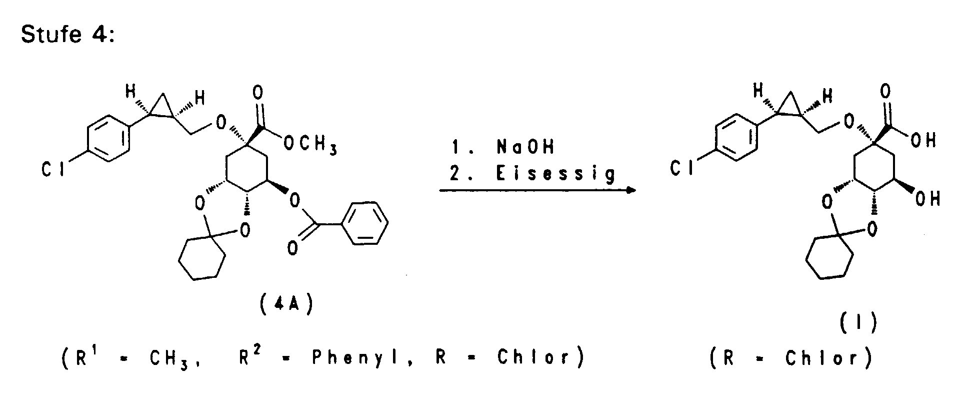
99.0 g (0.178 Mol) Verbindung 4A aus Stufe 3 wurden in 1200 ml Dioxan gelöst und 890 ml 2 N Natronlauge wurden zugesetzt. Die Suspension wurde 2 Stunden auf 80°C erwärmt.

Die Reaktionslösung wurde auf ca. 10°C heruntergekühlt und 228 ml (2 Mol) halbkonz. Eisessig langsam zugetropft (pH 5-6). Anschließend wurde die Lösung soweit einrotiert bis die erste Trübung auftrat. Dieses Konzentrat wurde unter kräftigem Rühren auf ca. 1500 ml Wasser gegossen, aus dem nach 10 Minuten Rühren ein kristalliner Niederschlag ausfiel. Dieser Niederschlag wurde abgesaugt und im Vakuum bei 22°C und 0.5 bar getrocknet. Man erhielt 82,2 g Verbindung I, R = Chlor.

### Beispiel 2

In analoger Weise wie in Beispiel 1 beschrieben erhält man die Verbindung der Formel

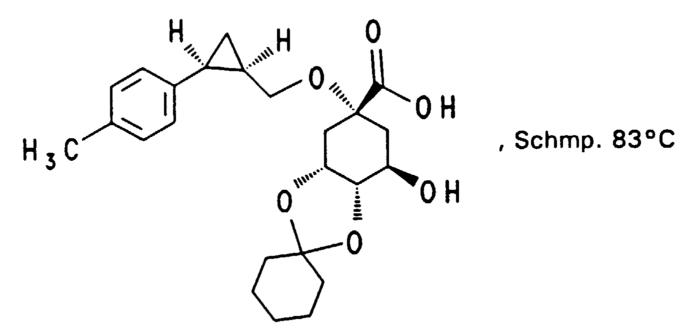

### Beispiel 3

In analoger Weise wie in Beispiel 1 beschrieben erhält man die Verbindung der Formel

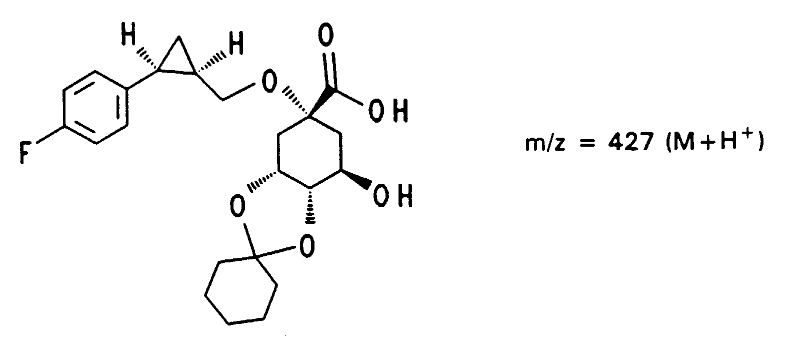

## Patentansprüche

1. Verfahren zur Herstellung einer Verbindung der Formel I 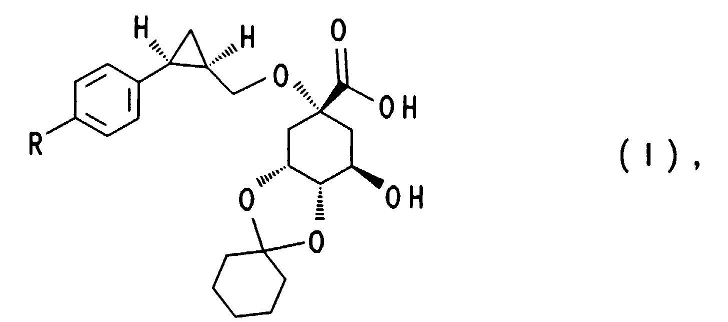 worin R Fluor, Chlor oder Methyl bedeutet, dadurch gekennzeichnet, daß man den Lactonring von 1,2-O-Cyclohexyliden-3,5-lactonyl-cyclohexan-1,2-diol der Formel 1 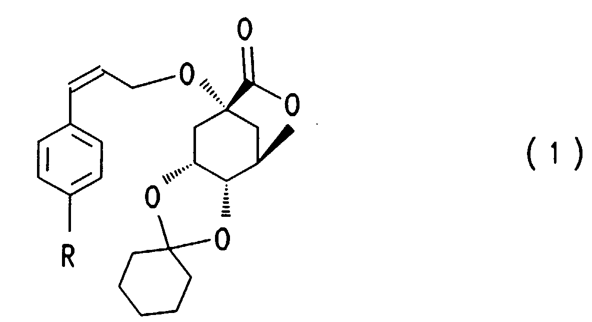 durch Basenkatalyse unter Bildung eines Alkohols der Formel 2 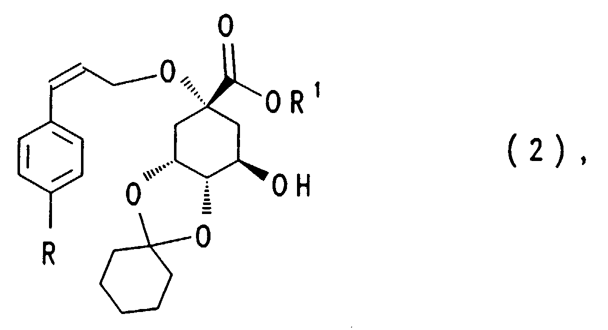 worin R die zu Formel I angegebene Bedeutung hat und R¹ C₁-C₁₀-Alkyl (geradkettig oder verzweigt) oder Benzyl bedeutet, öffnet,
einen erhaltenen Alkohol der Formel 2 mit einem Säurechlorid in Gegenwart einer Base umsetzt zu einem Ester der Formel 3 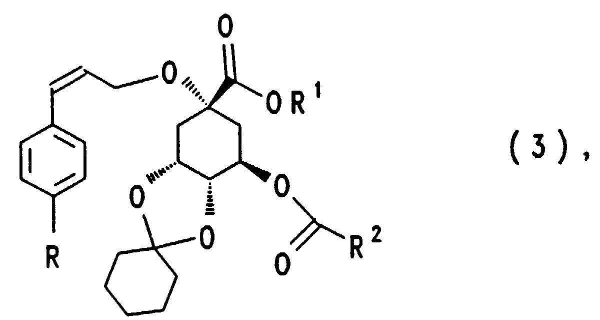 worin R die zu Formel I und R¹ die zu Formel 2 angegebenen Bedeutungen haben und
R² C₁-C₁₀-Alkyl (geradkettig oder verzweigt), Phenyl oder Naphthyl ist, und
eine erhaltene Verbindung der Formel 3 mit einem Carben umsetzt zu einem Diastereomerengemisch der Cyclopropylderivate der Formeln 4A und 4B 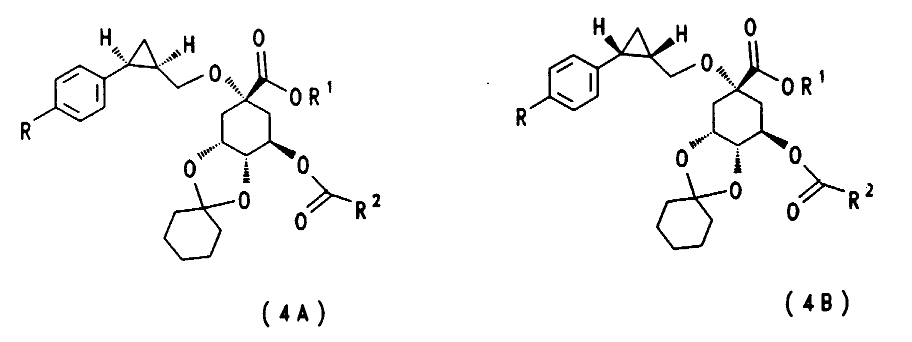 worin R, R¹ und R² die zu den Formeln I, 2 und 3 angegebenen Bedeutungen haben und wobei 4A in deutlichem Überschuß entsteht, durch Kristallisation eine Verbindung der Formel 4A aus dem Gemisch isoliert und durch alkalische Hydrolyse und anschließendes Ansäuern in eine Verbindung der Formel I überführt.

2. Verbindung der Formel I 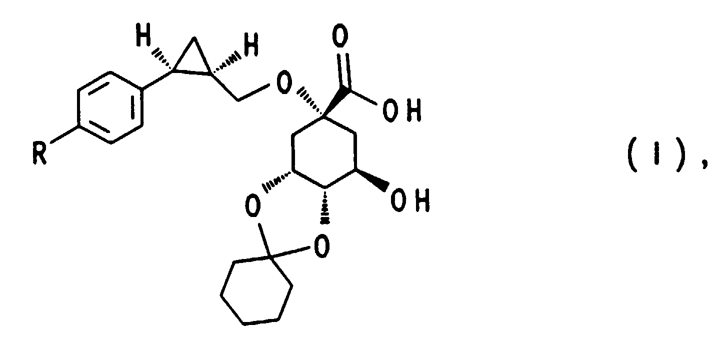 worin R Fluor, Chlor oder Methyl bedeutet.

3. Alkohol der Formel 2 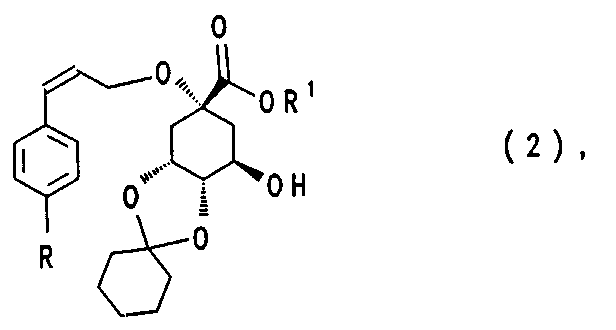 worin R Fluor, Chlor oder Methyl und R¹ C₁-C₁₀-Alkyl (geradkettig oder verzweigt) oder Benzyl bedeuten.

4. Ester der Formel 3 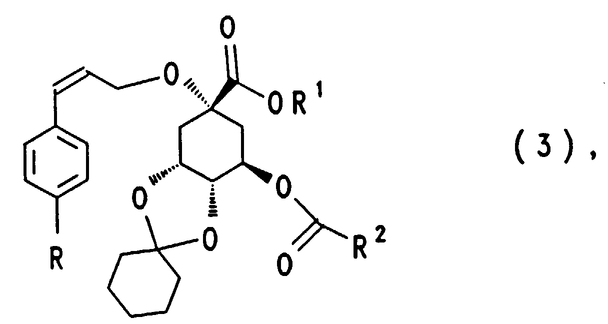 worin R und R¹ die zu Formel 2 in Anspruch 3 angegebenen Bedeutungen haben und
R² C₁-C₁₀-Alkyl (geradkettig oder verzweigt), Phenyl oder Naphthyl ist.

5. Cyclopropylderivat der Formel 4A 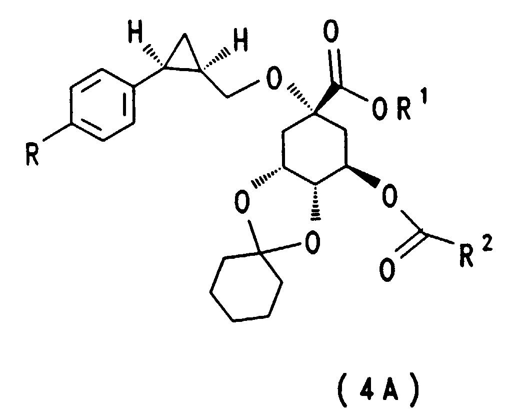 worin R, R¹ und R² die zu den Formeln 2 und 3 in den Ansprüchen 3 und 4 angegebenen Bedeutungen haben.

## Claims

1. A process for the preparation of a compound of the formula I 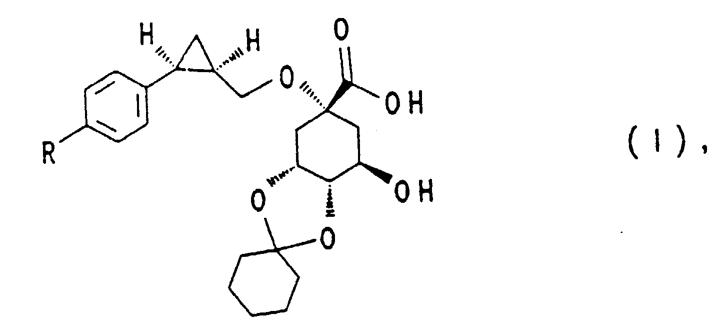 in which R is fluorine, chlorine or methyl, which comprises opening the lactone ring of 1,2-O-cyclohexylidene-3,5-lactonylcyclohexane-1,2-diol of the formula 1 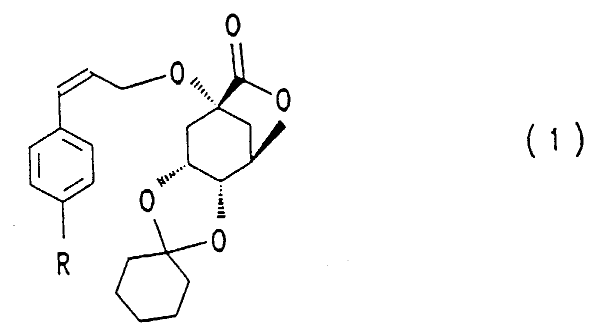 by base catalysis to form an alcohol of the formula 2 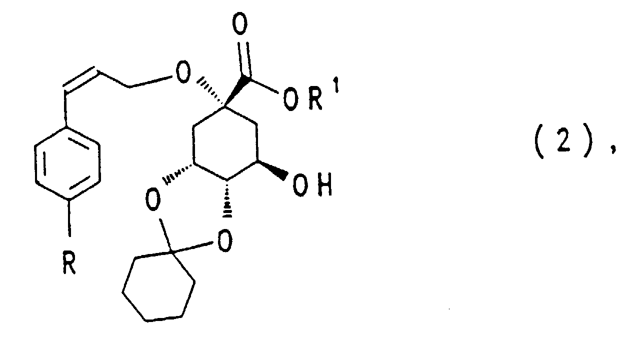 in which R is as defined for formula I and R¹ is C₁-C₁₀-alkyl (straight-chain or branched) or benzyl,
reacting a resulting alcohol of the formula 2 with an acid chloride in the presence of a base to give an ester of the formula 3 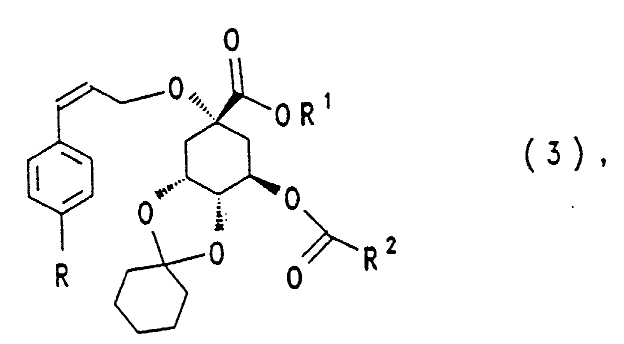 in which R is as defined for formula I and R¹ is as defined for formula 2, and
R² is C₁-C₁₀-alkyl (straight-chain or branched), phenyl or naphthyl, and
reacting a resulting compound of the formula 3 with a carbene to give a diastereomer mixture of the cyclopropyl derivatives of the formulae 4A and 4B 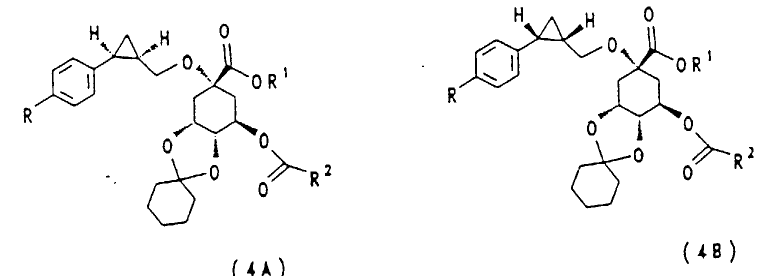 in which R, R¹ and R² are as defined for the formulae I, 2 and 3, in which 4A is present in a distinct excess, isolating a compound of the formula 4A from the mixture by crystallization and converting it into a compound of the formula I by alkaline hydrolysis followed by acidification.

2. A compound of the formula I 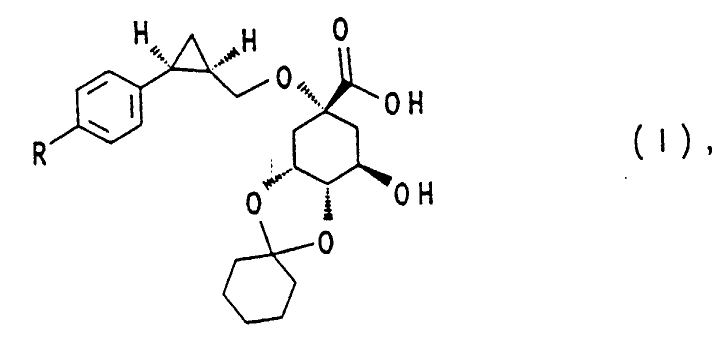 in which R is fluorine, chlorine or methyl.

3. An alcohol of the formula 2 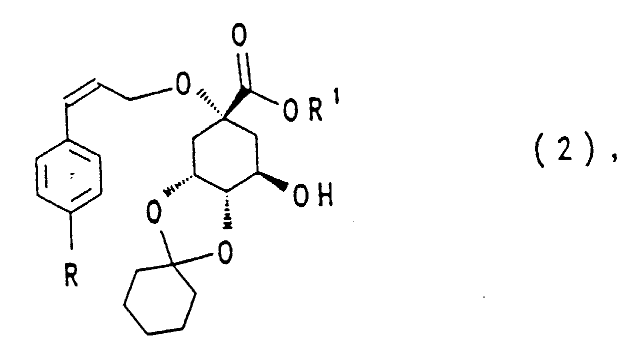 in which R is fluorine, chlorine or methyl and R¹ is C₁-C₁₀-alkyl (straight-chain or branched) or benzyl.

4. An ester of the formula 3 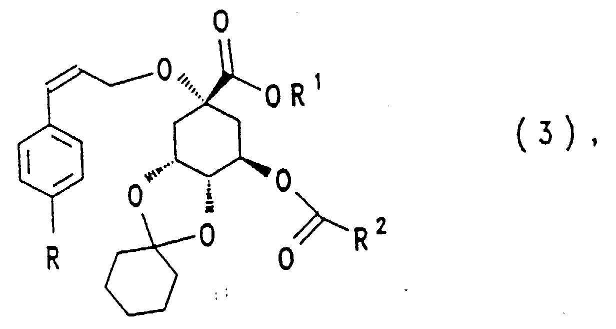 in which R and R¹ are as defined for formula 2 in claim 3, and
R² is C₁-C₁₀-alkyl (straight-chain or branched) , phenyl or naphthyl.

5. A cyclopropyl derivative of the formula 4A 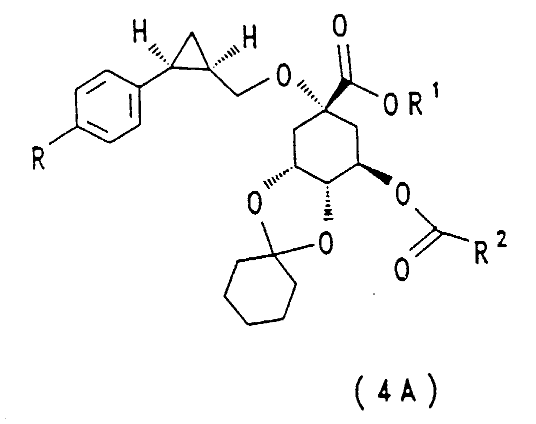 in which R, R¹ and R² are as defined for the formulae 2 and 3 in claims 3 and 4.

## Revendications

1. Procédé pour la préparation d'un composé de formule I 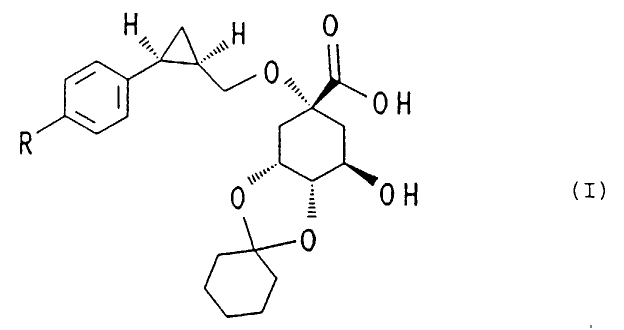 dans laquelle R représente un atome de fluor ou de chlore ou le groupe méthyle, caractérisé en ce que l'on ouvre le cycle lactone du 1,2-O-cyclohexylidène-3,5-lactonyl-cyclohexane-1,2-diol de formule 1 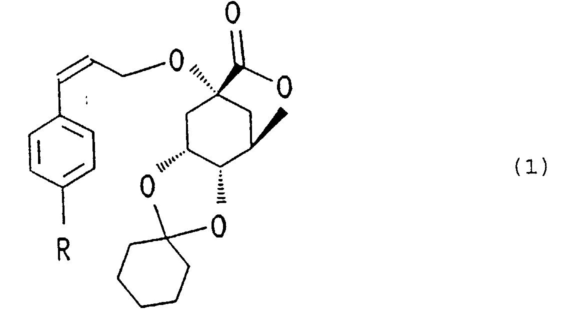 au moyen d'une catalyse par une base, avec formation d'un alcool de formule 2 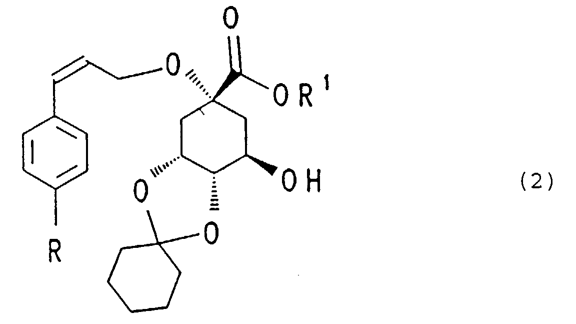 dans laquelle R a la signification donnée à propos de la formule I et R¹ représente un groupe alkyle en C₁-C₁₀ (à chaîne droite ou ramifié) ou le groupe benzyle,
on fait réagir l'alcool de formule 2 obtenu avec un chlorure d'acide, en présence d'une base, pour aboutir à un ester de formule 3 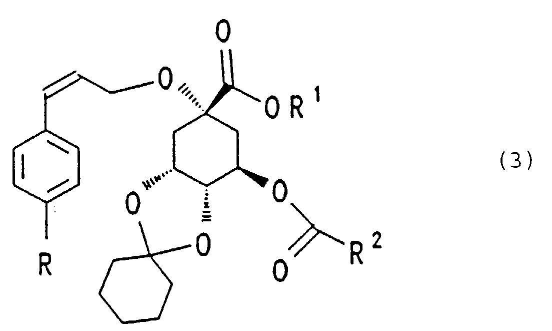 dans laquelle R a la signification donnée à propos de la formule I et R¹ a la signification indiquée à propos de la formule 2, et
R² est un groupe alkyle en C₁-C₁₀ (à chaîne droite ou ramifié), phényle ou naphtyle, et on fait réagir le composé de formule 3 obtenu avec un carbène, pour aboutir à un mélange de diastéréoisomères des dérivés cyclopropyle de formules 4A et 4B 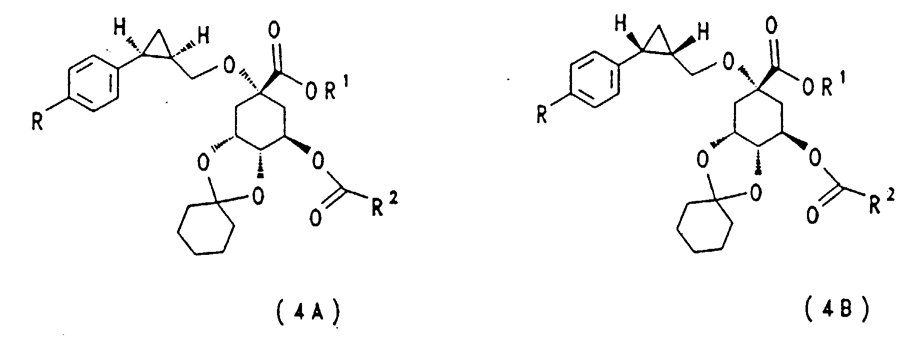 dans lesquelles R, R¹ et R² ont les significations données à propos des formules I, 2 et 3, et le composé 4A se formant en un net excès,
on isole par cristallisation un composé de formule 4A à partir du mélange, et on le convertit, par hydrolyse alcaline et acidification subséquente, en le composé de formule I.

2. Composé de formule (I) 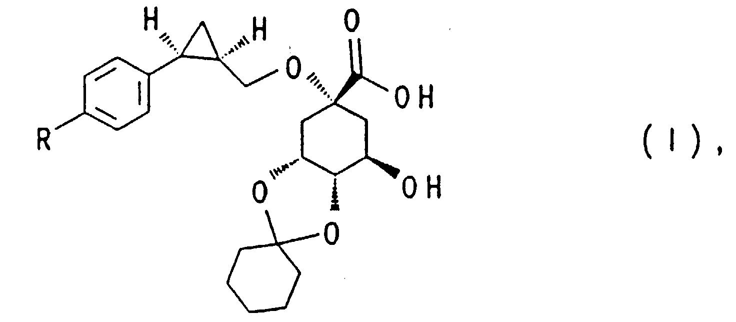 dans laquelle R représente un atome de fluor ou de chlore ou le groupe méthyle.

3. Alcool de formule 2 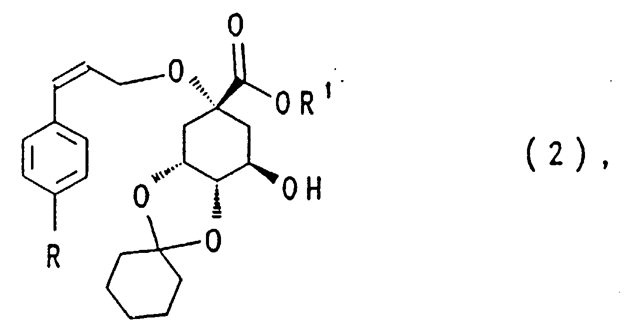 dans laquelle R représente un atome de fluor ou de chlore ou le groupe méthyle, et R¹ représente un groupe alkyle en C₁-C₁₀ (à chaîne droite ou ramifié) ou benzyle.

4. Ester de formule 3 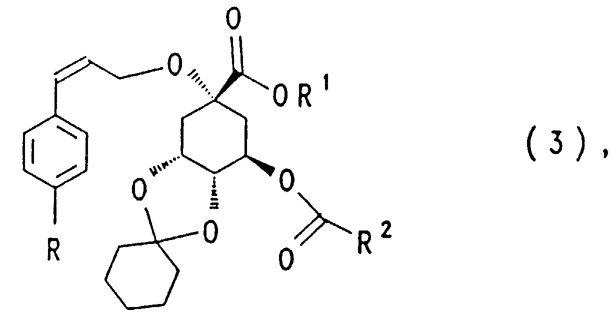 dans laquelle R et R¹ ont les significations données à propos de la formule 2 dans la revendication 3 et R² est un groupe alkyle en C₁-C₁₀ (à chaîne droite ou ramifié), phényle ou naphtyle.

5. Dérivé cyclopropyle de formule 4A 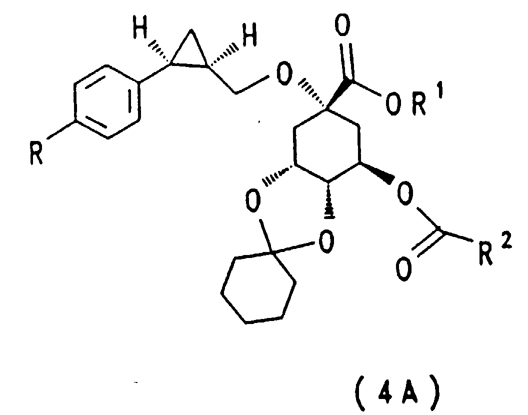 dans laquelle R, R¹ et R² ont les significations données à propos des formules 2 et 3 dans les revendications 3 et 4.
